# EUROPEAN PATENT SPECIFICATION

(11) **EP 1 649 060 B1**
(45) Date of publication and mention of the grant of the patent: **20.08.2008**
(21) Application number: 04776771.0
(22) Date of filing: 18.06.2004
(51) Int. Cl.: C12Q 1/68

(54) **MRNA EXPRESSION ANALYSIS**
MRNA-EXPRESSIONSANALYSE
ANALYSE D'EXPRESSION D'ARNM

(30) Priority: 27.06.2003 US 482980 P; 20.05.2004 US 851573
(43) Date of publication of application: 26.04.2006
(73) Proprietor: Biocept, Inc., San Diego, CA 92121 (US)
(72) Inventor: PIRCHER, Tony, J., San Diego, CA 92128 (US)
(74) Representative: HOFFMANN EITLE
(86) International application number: PCT/US2004/019559
(87) International publication number: WO 2005/001139

(56) References cited:
- WO-A-02/06511
- WO-A-97/05277
- WO-A-97/47640
- WO-A-98/10095
- WO-A-99/32663

## Description

### Field of the Invention

The present invention relates to methods for isolating and quantifying messenger RNAs (mRNAs) of interest that may be present in biological samples. The invention is believed to be particularly useful for mRNA expression analysis, but it will also have other molecular biological applications.

### Background of the Invention

The use of DNA microarrays to study gene expression has become increasingly popular; however, a number of complex steps which include enyzmatic reactions are now required to use the presently commercially available DNA analyses that employ microarrays. One of the steps is the conversion of unstable mRNAs into stable DNAs and RNAs, either with or without amplification of the mRNAs or of the resulting DNAs and RNA. A detectable entity or label, such as a fluorescent dye may be conveniently incorporated into the DNA during this enzymatic step. Labeled DNA targets are presently prepared from mRNAs in a number of ways. For example, in a typical experiment, the mRNAs in the sample are reacted with reverse transcriptase to produce labeled cDNA via in-situ incorporation of dye-labeled nucleotides or dye-labeled primers. Generally, such methods using enzymes result in the production of labeled targets that are heterogeneous in terms of their sizes and of the extent of dye incorporation. In addition, the efficiency of such enzyme reactions is dependent on environmental conditions, which may frequently be quite difficult to carefully control, resulting in an outcome that can be highly variable from sample to sample. It may be ideal if synthetic or biological targets designed by users are used to capture specific mRNAs quantitatively and to hybridize subsequently to a microarray.

Two such commonly used methods are the S1 nuclease protection assay, which is generally described in U.S. Patent No. 6,232,066, and the ribonuclease protection assay, which was described in Lee et al.; Methods in Enzymology, 152, p. 633-648 (1987). These assays use a number of different, fluorescently-labeled DNA targets, which are synthetically or biologically produced, to capture specific mRNAs. Thereafter, unbound labeled DNA targets and remaining mRNAs are digested using S 1 nucleases or ribonucleases, which are specific to single strand RNAs and to unbound DNA targets. A subsequent basic treatment is then employed to degrade the RNA portion of such double strands, leaving only the labeled synthetic targets that had hybridized to their respective specific mRNAs. Such targets can be hybridized, for example, with probes that are part of a relevant microarray for the detection and quantification of specific mRNAs. Although such methods would seek to ensure that the size of targets and the degree of dye incorporation are uniform and that all of the non-hybridized DNA targets and RNA have been digested so that there will be assured reproducibility in carrying out the final microassays, these methods usually rely on the use of the enzyme, S 1 nuclease, or the use of ribonucleases. As a result, small changes in reaction conditions may lead to highly variable amounts of the labeled targets due to either overdigestion or incomplete digestion.

WO 97/47640 discloses a method for mRNA analysis where mRNAs in a sample are selectively hybridised with biotinilated probes After immobilisation on a solid substrate, nuclease treatment digests non(perfectly)hybridised nucleic acids allowing the (quantitative) identification of specific(ally expressed) mRNAs in the sample by detection of the undigested immobilised probes.

In other non-related instances, magnetic beads have been used to isolate and purify mRNA, to construct solid-phase DNA libraries, and for PCR, differential display and subtractive hybridization applications. Magnetic beads, which are commercially available for these purposes are produced in various ways; often paramagnetic metals, such as metal oxides, are encapsulated with a suitable coating material, such as a polymer or a silicate, to produce coated beads that are about 1 µm -100 µm in diameter. These coating materials can be directly derivatized with oligo d(T), and magnetic beads with coatings derivatized with oligo d(T) have been used to isolate and purify mRNAs from total RNA or directly from tissues or cells after lysis. This method is effective because there is a tight binding affinity between oligo d(T) and poly A tails of mRNAs. Binding the mRNAs to a solid substrate, such as magnetic beads, and then performing additional reactions while they remain bound to the solid substrate offers many advantages over performing conventional solution-phase reactions. One advantage is easy washing between steps to remove unwanted materials. The coating material may also be derivatized with avidin, streptavidin or specific DNA sequences; in the case of streptavidin-derivatized beads, biotinylated oligo d(T) is used as an intermediate coupler to capture mRNAs. cDNA libraries have also been constructed by first capturing mRNAs with oligo d(T) coupled to magnetic beads. The bead-bound mRNA is then used as a primer for reverse transcriptase to synthesize first-strand cDNA, thus creating a covalently-linked first-strand cDNA library. Such a bead-bound cDNA library can be used as a template for PCR amplification. Differential display has also been carried out based upon selective reverse transcription of mRNAs and subsequent amplification using PCR. Magnetic beads have also been used to select mRNAs of interest through subtractive hybridization; however, they have not heretofore been considered to be helpful to analyze mRNA expression.

The aforementioned assay applications are heavily dependent upon using enzymes which inherently injects a degree of variability into any application, and the search has continued for improved mRNA expression analyses that can produce results that are truly stoichiometrically proportional to the amount of specific mRNAs in a biological sample and that are reproducible.

### Summary of the Invention

The present method provides multiple synthetic targets that are suitably labeled, as with a fluorescent dye, which targets are complementary to and stoichiometrically proportional to specific mRNAs that have been created without the involvement of enzymes and that are of precise composition. These labeled synthetic targets are constructed to facilitate, ultimate detection by hybridizing to complementary probes in a known format, such as a microarray, to allow subsequent quantification in a straightforward manner. To simplify and increase throughput of this mRNA expression analysis, the mRNAs in a biological sample are coupled to a solid support (e.g., streptavidin-derivatized magnetic beads) by preferably using biotinylated oligo d(T) which readily links to the beads and to the mRNA. Labeled targets specific to certain mRNAs of interest that are to be identified and quantified are added to a biological sample containing mRNAs and biotinylated oligo d(T). The mixture may originally contain the magnetic beads; however, the beads are preferably added after hybridization. After hybridization and attachment to the beads has been completed, unbound and non-specifically bound targets and non-mRNA material are removed by successive stringent washings. Next, all the mRNAs are eluted from the beads, as by subjection to conditions that separate the poly A and the oligo d(T), and the original mRNAs are then degraded by basic hydrolysis to leave labeled single-strand synthetic targets for further hybridization to their specific probes suitably carried on a microarray. Because both the probes and the targets are of synthetic designs, the size of the targets is easily normalized. In addition, because all affinity reactions, including hybridization reactions between the targets and the mRNAs, are finalized when the mRNA is bound to the solid substrate, purification of adducts can be performed by simple washings in combination with magnetic separation, as a result of which the need for any separation procedure based on column separation, filter preparation, centrifugation or precipitation, which can be laborious and time-consuming, is eliminated, thus rendering the analysis particularly attractive.

In one particular aspect, the invention provides a method for mRNA analysis, which method comprises providing a biological sample containing mRNAs, providing targets which are capable of selectively hybridizing to mRNAs of interest, associating said targets with said mRNAs under conditions conducive to hybridization, coupling mRNAs in the biological sample to a solid support, following passage of time sufficient for hybridization to occur, removing all unbound and non-specifically bound targets, degrading said mRNAs from the product following such removal to leave the targets that had earlier hybridized, and identifying the presence and quantity of particular targets by the use of known probes to which said targets selectively bind, as a result of which identification of the quantities of specific mRNAs in a biological sample can be accurately determined.

In another particular aspect, the invention provides a method for mRNA analysis, which method comprises providing a biological sample containing mRNAs, providing labeled targets which are capable of selectively hybridizing to mRNAs of interest, providing solid support material which carries oligo d(T), associating said labeled targets with said sample containing mRNAs and with said solid support material, maintaining such association under conditions conducive to, and for a time sufficient for hybridization to occur and for said mRNAs to couple to said solid support materials, thereafter washing said solid support material to remove all unbound and non-specifically bound targets and other unbound material, then releasing said coupled mRNAs and labeled targets from said solid support material, and separating same therefrom, nonenzymatically degrading said mRNAs from the product released from said solid support material to leave freed labeled targets that had earlier hybridized, and identifying the presence and quantity of particular labeled targets in said product by assaying for said targets, as a result of which identification of the quantities of specific mRNAs in the biological sample can be determined.

### Brief Description of the Drawings

FIG. 1 is a flow sheet showing a method for analysis of a biological sample embodying various features of the invention.
FIG. 2 is a schematic illustration showing an analysis procedure for a biological sample including mRNA which diagrammatically illustrates one preferred method of analysis embodying various features of the invention.

### Detailed Description of the Preferred Embodiments

The present invention describes methods for parallel, in vitro quantification of gnomic material, e.g. mRNAs, and it is considered to be particularly valuable for mRNA expression analyses. The method steps include the production of labeled synthetic single strand DNA targets which are complementary to specific mRNAs of interest. Such targets are preferably produced without the use of any enzymes and are provided in an amount at least stoichiometrically equivalent to the mRNAs of interest in a sample; they thus permit subsequently quantifying the mRNAs by hybridizing the labeled targets to probes on, for example, a microarray. This invention makes the task of identifying and quantifying the mRNA present in a biological sample simpler and more cost effective and generates more consistent results than present commercially available procedure.

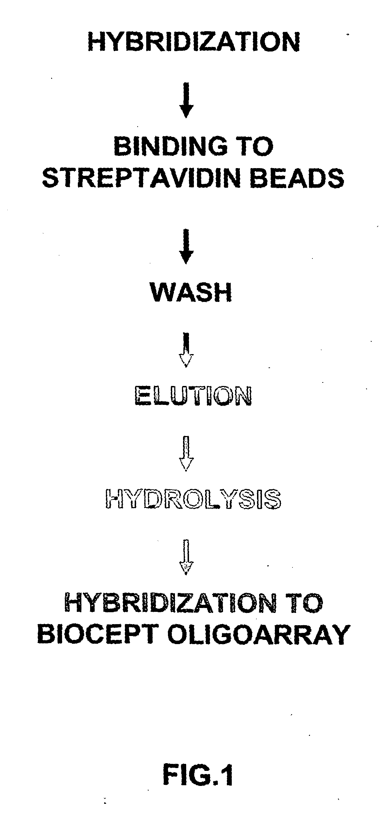

FIG. 1 is a flow sheet that provides a very brief overview of one preferred method of analysis embodying features of the invention. In accordance with practicing the method exemplified by FIG. 1, labeled targets, which are complementary to the mRNAs of interest, are mixed with the biological sample under conditions conducive to hybridization. Preferably coupling agents which carry oligo d(T) are added to the mixture at this time in order to couple with all of the mRNA in the sample, which agents are later used to couple to the solid support; alternatively such coupling to the mRNAs through their poly A tails may be deferred until after hybridization. After sufficient time for hybridization has passed, a plurality of magnetic beads are added to the mixture. Pursuant to the latter option, the coupling agents might be attached directly to the beads; however, the beads preferably carry binding agents or linkers, e.g. streptavidin, that are complementary to linkers carried by oligo d(T) coupling agents earlier mixed with the sample. After providing time for the attachment between the beads and the mRNA to be completed, the beads are washed to eliminate all of the other components of the biological sample and to remove the labeled targets that have not specifically hybridized to mRNA.

Generally, one or more additional washings, optionally of increased stringency, will be employed in order to remove any labeled target that may hybridize loosely to a sequence in the mRNA not precisely that of a desired nucleotide sequence. Following these washings, the mRNA is eluted from the solid support by breaking a bond between the mRNA and the solid support, as described in detail hereinafter. Following elution, the liquid supernatant is separated from the beads which are being immobilized by magnetic attraction, and it is then subjected to hydrolysis by chemical treatment. All of the natural RNA, including that hybridized to the labeled targets, is degraded; following this hydrolysis, the only molecules still intact in the liquid solution are the labeled targets. The hydrolyzed mixture can then be directly subjected to any suitable microassay, as the presence of the degraded material does not interfere with such a microassay. Accordingly, if the labels are fluorescent, the signals that will be given off by probes that are specific to the targets that were originally mixed with the biological sample will provide a quantitative indication of the relative amount of that particular mRNA which was present in the biological sample.

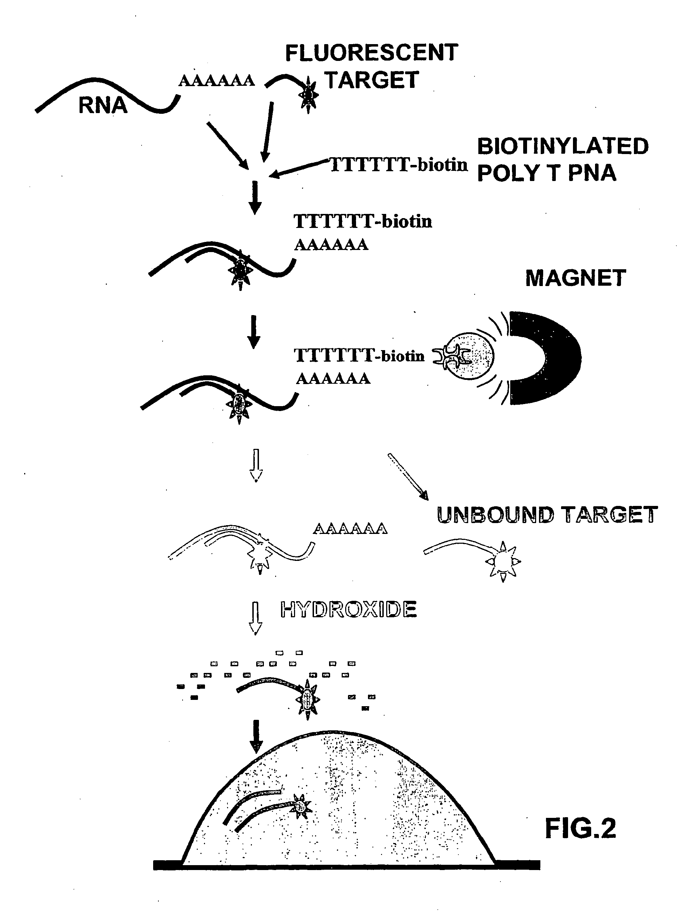

Illustrated in FIG. 2 is a preferred assay method wherein labeled single-strand DNA targets are provided which are designed to selectively hybridize to mRNAs of interest for the purpose of quantitatively determining the concentration of such mRNAs in a biological sample. These items referred to as labeled targets may have one of a variety of suitable forms known in the art for hybridizing to mRNA; however, preferably, the targets are synthetic oligonucleotides that will be of suitable length between about 10 and 100 nucleotides and preferably between about 30 and 60 nucleotides in length, having sequences that will be complementary to particular nucleotide sequences of each mRNA of interest. The targets are preferably formed on automatic DNA synthesizers; however other known methods may alternatively be employed. Although natural nucleotides are often used, peptide nucleic acids (PNAs) or other non-natural synthetic nucleotides might be alternatively employed to increase affinity. The oligonucleotides target may be connected directly to a label, but often a short linker is used so the label will not interfere with hybridization of the target to the mRNA, as is well known in this art. Although for simplicity purposes, the DNA oligonucleotides targets will be the moieties to which complementary probes in the final assay will be constructed, alternatively each target may include a second DNA section that would serve as a unique identifier that is linked as a second part of the overall labeled target; in such an instance, the final assay would have probes specific for each particular unique identifier employed in a particular assay at a particular microspot for that assay. Thus, the presence of a signal on a microarray or the like at that microspot would be evidence of the presence of the corresponding linked target that is complementary to a specific mRNA of interest in the biological sample.

The labels used may be any of those items that have commonly been used, selected from the wide range of materials commercially available for labeling nucleic acids, including indicator dyes, radionuclides, antibodies, enzymes and the like. Preferably, the label is a fluorescent dye for simplification of the final assay; however alkaline phosphates, peroxides, β-galactosidase (beta-galactosidase) and haptens, such as digoxin and digoxygenin, as well as items as chemiluminescent moieties may be used.

As indicated above, although the use of oligo d(T) linked to the solid support is the preferred method of coupling the mRNA in the biological sample to the solid support, other alternative methods of coupling might be employed. For example, instead of using oligo d(T), short nucelotide sequences might be chosen that would be found in the mRNAs of interest in regions outside of the region where the hybridization with the target material will occur. These shorter stretches of oligonucleotides might also be found in other mRNAs, but this would not be a deterrent. On balance, it is believed that use of oligo d(T) for coupling the solid support material to the mRNAs is by far preferred, and such an arrangement simplifies the overall expression assay.

The solid support material can be selected from any of a wide variety of materials that are commonly used, such as those which are commercially available from Amersham Biosciences, BioRad, and Sigma. It can be in the form of particles, plates, matrices, fibers or the like, and it may be made of silica, cellulose, agarose beads, controlled-pore glass, polymeric beads, gel beads, or magnetic beads. Magnetic beads are preferred because the use of such facilitates their subsequent separation from the supernatant by the straightforward application of a magnetic field. Such can be done using flow chambers or by simply pipetting. Such magnetic beads, for example those sold as Dynal beads or those sold by Advanced Magnetics as BIO-MAG beads, can be used to separate the coupled mRNA from the remainder of the biological sample and the unbound and non-specifically bound target material as a part of an original washing. The same property is also taken advantage of in separating the decoupled mRNA at a later stage in the assay. Although the particles in bead form are preferred for facility and handling, other shaped particles or substrates might alternatively be employed. Such commercially available magnetic beads are generally small nonporous spheres that are coated with a layer of magnetite to provide the desired magnetic properties, and then with an exterior coating.

To couple the mRNAs to the beads, various arrangements can be employed. For example, if oligo d(T) is to be used to attract and couple the mRNAs through their poly A tails, oligo d(T) can be linked directly to the surface of the beads, or to such other solid support that is being used. Magnetic beads having oligo d(T) essentially linked to its surface is commercially available.

The preferred arrangement, however, employs, first and second linkers or binding agents which are complementary and bind to each other as an intermediate linkage to attach the coupling agent, i.e. oligo d(T), with the solid support. Many varieties of binding pairs are well known in the art and may be suitably employed. A preferred binding system employs avidin or streptavidin and biotin. Streptavidin, for example, is covalently attached to the exterior surface of the solid support, e.g., the magnetic beads, and it, in turn, binds strongly to biotinylated oligo d(T). Such magnetic beads suitable for applications of interest are commercially available from a number of vendors. Beads which have streptavidin bound to the surface of the beads, having a nominal size of about 1 micron in diameter, are sold by Active Motif of Carlsbad, California. Other binding pairs, e.g. antibody-antigen and the like, may alternatively be used as such an intermediate linkage.

Although biotinylated oligo d(T) comprising natural thymine may be employed as the coupling agent to couple to the mRNA, stronger coupling can be obtained through the use of synthetic oligo d(T) which includes RNAs or other non-natural nucleotides of various forms that are more strongly attracted to the poly A tails of the mRNAs. As a result, this arrangement allows more aggressive washing to remove non-specifically bound labeled targets and thus provides a cleaner assay as described in more detail hereinafter. Such biotinylated oligo d(T) using non-natural nucelotides is commercially available; one product is that sold as Poly T grip NA Probe by Active Motif, which is a PNA oligomer of thymine replicas and which carries a biotin molecule at the 5' end. Such an arrangement using commercially available materials facilitates implementing the assay procedure step wherein the mRNA is sequestered from the biological sample by the solid support material preparatory to separation of unbound material.

The steps in the initial portion of the assay can be performed in various sequences. For example, the biotinylated oligo d(T) might be first mixed with the biological sample containing the mRNAs before the labeled targets are added to the mixture, or the biotinylated oligo d(T) might be first coupled with the streptavidin-carrying magnetic beads before being mixed with the biological sample (and also optionally washed to remove non-mRNA material from the sample before contact with the target material). However, the preferred method of carrying out the assay is depicted in FIG. 2 wherein a mixture is first made containing the biological sample, which includes the mRNA being analyzed, the labeled targets, i.e. DNA oligonucleotides which carry fluorescent labels, and the biotinylated oligo d(T), ' preferably including PNAs. This mixture is maintained under conditions conducive to hybridization so that the oligo T PNA links to the poly A tails of all of the mRNA available, and so that the single strand DNA targets hybridize to the specific sections of the mRNA containing the precise complementary sequences, assuming such are present in the sample. Hybridization is preferably carried out in the presence of a hybridization buffer, at a pH of about neutral, with appropriate salt concentrations and at a temperature of about 20° to 45° C for about 3 hours to 24 hours (i.e., overnight).

Once the time desired to assure essentially complete hybridization has passed, streptavidin-carrying magnetic beads are added to the mixture. The beads have preferably been pre-blocked overnight with 0.2% BSA solution, as well known in this art, before they are added to the mixture containing the biological sample. Thereafter, incubation is continued at about 20° to 45°C, with constant agitation of the beads within the liquid mixture, for about 30 minutes to one hour and preferably for about 45 minutes. Under these conditions, it is expected that essentially all of the biotinylated material will have become attached to the magnetic beads as a result of the binding of the biotin-streptavidin pairs.

Once this time period has passed, washing is carried out in order to remove all non-mRNA material from the biological sample and all unbound and non-specifically bound labeled targets. Generally, multiple washings will be carried out that may optionally use washing solutions of increasing stringency, as well known in this art. As previously mentioned, the employment of such non-natural thymine as part of the poly T couplers allows high stringency washings, without significant danger of inadvertently eluting some of the mRNAs to which targets have hybridized. By the use of tiny magnetic beads, e.g. about 1 micron diameters, as the solid support, such washing is facilitated because the beads can be effectively agitated and then held against a wall of a chamber, e.g. a test tube of suitable size, following agitation while the supernatant is removed by pipetting or the like. Alternatively, the beads can be transferred to a flow chamber where they are suitably alternately agitated and then magnetically restrained while solutions of higher and higher stringency are caused to flow through the chamber to effect the desired washings.

Once the desired washing is complete to remove all labeled targets not specifically bound, the mRNA is preferably eluted from the beads, prior to the following step of degradation. Although degradation could be carried out while the mRNA remains coupled to the solid support, inasmuch as it is desirable to separate the target material from the beads prior to a final assay on a microarray or the like, it is preferred to carry out the separation at this point. Elution is carried out by suitably breaking the bond between the poly A tails of the mRNA and the oligo d(T) by heating to about 75°C in deionized (DI) water, and then separating the supernatant from the beads while at such temperature.

With the beads removed, the mRNA mixture in the DI water is treated with alkali, e.g. a sodium hydroxide solution, for about 15 minutes at a suitable temperature, e.g. RAT to 75°C, to degrade the mRNAs by hydrolysis. This frees the fluorescent-labeled synthetic targets, which as previously indicated, are preferably DNA oligonucleotides that remain unaffected by the alkali treatment. The resulting DI water solution can then be directly used in a suitable assay for the particular ' targets that were originally mixed with the biological sample and that have now been liberated as a result of the hydrolysis step.

Although any of the myriad of developed assays for labeled DNA targets can be used, including those two-dimensional assays wherein probes for targets are bound directly to a flat substrate, e.g; in a well of microtiter plate, assaying by using a three-dimensional biochip, such as those described in U.S. Patent No. 6,174,683 and in published international application WO 02/059372, entitled "Three Dimensional Format Biochips," is preferred. In such a three-dimensional assay, the probes are not connected to the solid surface of the well of a plate or to a glass slide or to a plate, but they are instead presented in three-dimensional array by attachment to microspots of polymerized hydrogel; this isolates the probes from the solid substrate and presents an expanded surface area for presentation of the probes and for the ultimate capture of the labeled target molecules. Accordingly, a plurality of microspots are provided on each glass slide or in each well of a microwell plate, generally in proportion to the number of different targets employed in the expression assay. For example, if 50 targets are employed, then 50 microspots, i.e. an array of 5 x 10, might be provided on the surface of the glass slide or the like.

Following incubation of the hybridization solution with the slides or wells, washing is carried out to remove unbound labeled target material. The resulting slide can be observed in any suitable manner, as by using a fluorescence detector. Other appropriate detectors would of course be alternatively used depending on the nature of the particular label chosen for attachment to the targets.

mRNA expression assays embodying features of the invention, as explained above, avoid the use of enzymes in their procedures and thus eliminate a substantial potential for variability in the results depending upon enzymatic operating conditions and performance. The assays are not only simple and straightforward to perform, but by using the preferred alternatives, they are reliable and capable of producing fully reproducible results. Thus use of these assays can provide precise quantitative analyses of the amounts of particular mRNAs present in a biological sample.

The specifics of the invention will be further clarified by the following descriptions of several examples designed to focus upon particular features of the invention; however it should be understood that these examples are only for purposes of illustration and do not constitute limitations upon the scope of the invention which is of course defined by the claims that appear at the end of this application.

### Example 1

A mRNA assay is carried out using human total RNA and using 20 different targets constructed to be complementary to RNA that should be present and five negative control sequences that are constructed so as not to specifically hybridize to any natural RNA sequences that should be found in human RNA. Each of the targets is synthesized using natural nucleotides and is a 45-mer. Six nanomoles (nM) of each of the targets are used and combined with about 50 µg of human total RNA in a suitable test tube. Oligo d(T) at a concentration of 20 µM in a hybridization buffer (10 mM Tris, pH 7.5, 300 mM NaCl, 10 mM MgCl₂) is added. Each of the 45-mer targets is labeled with Cy-3, a fluorescent cyanine dye.

The mixture is allowed to hybridize at about 37°C overnight and then streptavidin-conjugated magnetic beads (one µm diameter) purchased from Active Motif, are added to the test tube holding the mixture. The beads are preblocked overnight with 0.2% BSA solution (10 mM HEPES, pH 7.3, 100 mM NaCl, 0.1% Triton X-100) at 4 °C. The resulting mixture is incubated at 37 °C for 45 minutes with constant agitation. The tube containing the mixture is then placed against magnetic stand to focus the beads to one wall, and supernatant is removed by a pipette. The tube is filled immediately with washing buffer (20 mM Tris, pH 7.5, 50 mM NaCl), and the resulting solution is gently agitated by pipetting up and down several times. The tube is then again placed against the magnetic stand, the supernatant is removed, and the tube is again filled with the washing buffer. The tube is then put on a rocker for 15 minutes to facilitate washing. The washing step is then repeated one more time. After the third wash, the supernatant is replaced with deionized (DI) water to elute bound mRNAs heating at about 75°C for 2 minutes, and the mixture is separated from the beds at this temperature. To the mRNA mixture in DI water, sodium hydroxide solution is added to provide an NaOH concentration about 0.1 molar; the mixture is maintained at about 75° C for about 15 minutes to degrade mRNA and thereby free all the Cy-3 labeled synthetic targets.

The resultant alkali solution containing the mRNA is neutralized with a 2M HEPES pH 5.3 buffer, followed by addition of 20xSSC (final to 3xSSC) and 4% Triton X-100 (final to 0.1%). It is then used directly with a standard microarray where microspots are provided on a glass slide at 25 locations. Each of the 25 mircrospots contains probes complementary to one of the 45-mers that were included in the original target solution. After overnight hybridization at about 45° C, the slide is washed to remove unbound target material from the region of each of the 25 microspots and then examined using a laser scanner (ScanArray Lite, Packard Biochip Technologies). Examination shows that the 20 microspots having probes directed to targets expected to be present in total human mRNA light up in about the brightness expected, whereas the five microspots having the probes complementary to the negative control 45-mers do not light up. As a result, the mRNA assay procedure is felt to be effective as an analysis tool of carrying out mRNA expression analyses and the like.

### Example 2

To provide a test sample, cRNAs were created for the human genes pdha, hprt1 and rb1. To mimic RNA, a polyA tail was added to the 3' end of each using Ambion's polyA tailing kit. A set of 20 different flourescent dye-labeled 45-mer oligonucleotides targets (6 nM final volume for each primer) was mixed with 5 µL of each of the three poly A cRNA targets (the final concentration was estimated to 1 ng), 50 µg human total liver RNA and binding buffer to yield a final 200 µL hybridization volume, and hybridization was then performed for about 4 hours at 37°C. The hybridization mixture was incubated with biotinylated poly T PNA overnight. The next day, streptavidin-coated magnetic beads were added to the hybridization mixture and agitated for 45 minutes at 37°C. Bound RNA and targets hybridized to the RNA were isolated using a magnetic stand, and the same washing and elution procedure as in Example 1 was then carried out. The supernatant was extracted from the beads as in Example 1, and sodium hydroxide was added to a similar concentration to degrade the RNA, leaving intact the 45-mer DNA targets with their fluorescent labels.

The resulting solution was applied to a glass slide made with three-dimensional microdroplets in accordance with the teachings of the aforementioned International Publication. After providing time for hybridization and then washing as in Example 1, the array was laser-scanned. Specific signals were detected for hprt1, pdha and rb1, demonstrating through such capture that specific hybridization and binding of those targets to the polyA-added cRNA templates that were created was obtained in the test assay.

### Example 3

To show that the assay procedure is effective to measure small changes in gene expression and to be able to reproducibly make such measurements, some additional tests are performed using 40 micrograms of human total kidney RNA as a template for each assay. A 200µL prehybridization mix was created that included 40µg human total kidney RNA, 6.25nM of each Cy3-labeled 45-mer primer, and binding buffer (300mM NaCl, 10mM MgCl2, 10 mM Tris pH 7.5). After short denaturation at 94°C, 5 µL poly T PNA was added to the mix, and thereafter 3 µL of RNaseOut (Invitrogen) is added to inhibit possible traces of RNase. The hybridization was performed overnight at about 37°C, and the next day 30 µL of streptavidin-bound magnetic beads (Active Motif) were added to each sample and agitated for 45 min at 37°C. After 3 washes at room temperature with low salt wash buffer (20 mM Tris, pH 7.5, 50mM sodium chloride), the mRNA including the specifically hybridized Cy3-labeled targets were eluted as in Example 1; thereafter, the mRNA was degraded with sodium hydroxide as in Example 1. The resulting labeled targets were hybridized to microarrays and analyzed. The variation in obtained signals during the three experiments is very small, i.e., the overall average coefficient of variation (CV) was about 7 percent.

Although the invention has been described with regard to certain preferred embodiments, which constitute the best mode for carrying out the invention as presently known, it should be understood that various changes and modifications to the various steps and materials described may be made as would be obvious to one having ordinary skill in this art without departing from the scope of the invention, which is defined in the claims appended hereto. For example, other binding agent pairs than biotin and streptavidin might be used. Moreover, when any such pairs are used, the oligo d(T) may optionally be linked to the solid support material prior to coupling with mRNAs in the biological sample. Alternatively, all of the initial components may be simultaneously blended as a mixture before being subjected to hybridization conditions.

Particular features of the invention are emphasized in the claims that follow.

## Claims

1. A method for mRNA analysis, which method comprises:
(a) providing a biological sample containing mRNAs,
(b) providing targets which are capable of selectively hybridizing to mRNAs of interest and which are resistant to degradation by basic hydrolysis,
(c) associating said targets with said mRNAs under conditions conducive to hybridization,
(d) coupling mRNAs in the biological sample to a solid support,
(e) following passage of time sufficient for hybridization of said targets and complementary sequences of mRNAs to occur, washing to remove all unbound and non-specifically bound targets and non-mRNA biological material in said biological sample,
(f) degrading said mRNAs from the hybridized product of step (e) by nonenzymatic treatment to leave the targets that had earlier hybridized, and
(g) identifying the presence and quantity of particular targets by the use of known probes to which said targets selectively bind, as a result of which identification of the quantities of specific mRNAs in a biological sample can be accurately determined.

2. The method according to claim 1 wherein said mRNAs are coupled to said solid support through the polyA tails of said mRNAs .

3. The method according to either claim 1 or 2 wherein oliqo d(T) which hybridizes to the polyA tails of said mRNAs is linked to said solid support.

4. The method according to claim 3 wherein said oligo d(T) individually carries first binding agents and wherein said solid support carries second binding agents of a character so that said first and second binding agents bind to each other to attach said oligo d(T) to said solid support.

5. The method according to any one of claims 1 to 4 wherein said degrading of said mRNAs from the hybridized product releases said targets and leaves them free from any connection to said solid support.

6. The method according to claim 5 wherein said released targets are then identified by hybridization to a microarray in the presence of other products of said degrading.

7. The method according to any one of claims 1 to 6 wherein said mRNAs in said biological sample are allowed to hybridize with said targets prior to said coupling of said mRNAs to said solid support.

8. The method according to any one of claims 1 to 7 wherein prior to step (f), said coupled mRNAs and hybridized targets are released from said solid support.

9. The method according to any one of claims 1 to 8 wherein said solid support comprises a plurality of magnetic beads which carry oligo d(T) and wherein said beads are mixed with the biological sample to couple the mRNAs thereto.

10. The method according to any one of claims 1 to 9 wherein said targets are single strand DNA oligonucleotides that directly hybridize to said mRNAs.

11. The method according to any one of claims 1 to 10 wherein said mRNAs are degraded by alkaline treatment in step (f) to leave said targets.

12. The method according to claim 11 wherein step (g) is carried out without separating said targets from the remains of degradation step (f).

13. The method according to any one of claims 1 to 12 wherein said targets are initially labeled and said labels are detected in step (g).

14. The method according to claim 13 wherein, as a part of step (g), the product of step (f) is separated from said solid support and hybridized with a microarray having a plurality of microspots of a three-dimensional character, at least one of which microspots contains probes complementary to each of said targets.

## Patentansprüche

1. Verfahren zur mRNA-Analyse, umfassend:
(a) Zurverfügungstellen einer biologischen Probe enthaltend mRNAs,
(b) Zurverfügungstellen von Zielsequenzen, die in der Lage sind, selektiv an mRNAs von Interesse zu hybridisieren und welche resistent sind gegenüber dem Abbau durch basische Hydrolyse,
(c) Assoziieren von besagten Zielsequenzen mit besagten mRNAs unter Bedingungen, die für die Hybridisierung geeignet sind,
(d) Koppeln von mRNAs in der biologischen Probe an einen festen Träger,
(e) nachdem ausreichend Zeit zum Hybridisieren von besagten Zielsequenzen und komplementären Sequenzen der mRNAS verstrichen ist, Waschen, um alle ungebundenen und nicht spezifisch gebundenen Zielsequenzen und nicht-mRNA biologisches Material in der besagten biologischen Probe zu entfernen,
(f) Degradieren von besagten mRNAs von dem hybridisierten Produkt von Schritt (e) durch nichtenzymatische Behandlung, um die Zielsequenzen überzulassen, die zuvor hybridisiert hatten, und
(g) Identifizieren des Vorliegens und der Menge von bestimmten Zielsequenzen unter Verwendung von bekannten Sonden, an welch besagte Zielsequenzen selektiv binden, durch welche Identifikation der Mengen von spezifischen mRNAs in einer biologischen Probe akkurat bestimmt werden können.

2. Verfahren gemäß Anspruch 1, worin besagte mRNAs an besagten festen Träger durch die PolyA-Schwänze von besagten mRNAs gekoppelt sind.

3. Verfahren gemäß Anspruch 1 oder 2, worin Oligo d(T), welches an die PolyA-Schwänze von besagten mRNAs hybridisiert, an besagten festen Träger gekoppelt ist.

4. Verfahren gemäß Anspruch 3, worin besagtes Oligo d(T) individuell erste Bindungsmittel trägt und worin besagter fester Träger zweite Bindungsmittel trägt, mit der Eigenschaft, daß besagte erste und zweite Bindungsmittel aneinander binden, um besagtes Oligo d(T) an besagtem festem Träger zu befestigen.

5. Verfahren gemäß einem der Ansprüche 1 bis 4, worin besagte Degradierung von besagten mRNAs von dem hybridisierten Produkt die besagten Zielsequenzen freisetzt und sie frei von jeglicher Verbindung mit besagtem festem Träger läßt.

6. Verfahren gemäß Anspruch 5, worin die besagten freigesetzten Zielsequenzen durch Hybridisierung an einen Mikroarray in der Gegenwart von anderen Produkten von besagtem Degradieren identifiziert werden.

7. Verfahren gemäß einem der Ansprüche 1 bis 6, worin besagte mRNAs in besagter biologischer Probe hybridisieren gelassen werden mit besagten Zielsequenzen vor dem besagten Koppeln von besagten mRNAs an den besagten festen Träger.

8. Verfahren gemäß einem der Ansprüche 1 bis 7, worin vor dem Schritt (f) die besagten gekoppelten mRNAs und hybridisierten Zielsequenzen von dem besagten festen Träger freigesetzt werden.

9. Verfahren gemäß einem der Ansprüche 1 bis 8, worin besagter fester Träger eine Vielzahl von magnetischen Kügelchen umfaßt, welche Oligo d(T) tragen, und worin besagte Kügelchen mit der biologischen Probe gemischt werden, um mRNAs daran zu koppeln.

10. Verfahren gemäß einem der Ansprüche 1 bis 9, worin besagte Zielsequenzen einfachsträngige DNA-Oligonukleotide sind, die direkt an besagte mRNAs hybridisieren.

11. Verfahren gemäß einem der Ansprüche 1 bis 10, worin besagte mRNAs durch alkalische Behandlung in Schritt (f) abgebaut werden, um besagte Zielsequenzen überzulassen.

12. Verfahren gemäß Anspruch 11, worin Schritt (g) ausgeführt wird ohne Auftrennen von besagten Zielsequenzen von den Hinterlassenschaften des Degradationsschritts (f).

13. Verfahren gemäß einem der Ansprüche 1 bis 12, worin besagte Zielsequenzen anfänglich markiert sind und besagte Marker in Schritt (g) detektiert werden.

14. Verfahren gemäß Anspruch 13, worin als Teil von Schritt (g) das Produkt von Schritt (f) aufgetrennt wird von besagtem festem Träger und hybridisiert mit einem Mikroarray, der eine Vielzahl von Mikrospots mit einem dreidimensionalen Charakter aufweist, wobei zumindest einer der Mikrospots Sonden enthält, die zu jeder der besagten Zielsequenzen komplementär sind.

## Revendications

1. Méthode d'analyse d'ARNm, méthode qui comprend les étapes consistant à :
(a) fournir un échantillon biologique contenant des ARNm ;
(b) fournir des cibles qui sont capables de s'hybrider sélectivement aux ARNm intéressants et qui sont résistantes à la dégradation par hydrolyse basique ;
(c) associer lesdites cibles auxdits ARNm dans des conditions conduisant à une hybridation ;
(d) coupler les ARNm présents dans l'échantillon biologique à un support solide ;
(e) après écoulement d'un temps suffisant pour que l'hybridation desdites cibles et desdites séquences complémentaires d'ARNm se produise, effectuer un lavage pour éliminer toutes les cibles non liées et liées non spécifiquement et les substances biologiques ne consistant pas en ARNm présentes dans ledit échantillon biologique ;
(f) dégrader lesdits ARNm du produit hybridé de l'étape (e) par traitement non enzymatique pour laisser les cibles qui ont été hybridées antérieurement ; et
(g) identifier la présence et la quantité de cibles particulières en utilisant des sondes connues auxquelles lesdites cibles se lient sélectivement, identification en résultat de laquelle les quantités d'ARNm spécifiques dans un échantillon biologique peuvent être déterminées avec précision.

2. Méthode suivant la revendication 1, dans laquelle lesdits ARNm sont couplés audit support solide par les queues polyA desdits ARNm.

3. Méthode suivant la revendication 1 ou 2, dans laquelle de l'oligod(T) qui s'hybride aux queues polyA des ARNm est lié audit support solide.

4. Méthode suivant la revendication 3, dans laquelle ledit oligod(T) porte individuellement des premiers agents de liaison et dans laquelle ledit support solide porte des seconds agents de liaison ayant des caractéristiques telles que lesdits premier et second agents de liaison se lient l'un à l'autre pour la fixation dudit oligod(T) audit support solide.

5. Méthode suivant l'une quelconque des revendications 1 à 4, dans laquelle ladite dégradation desdits ARNm du produit hybridé libère lesdites cibles et les laissent dépourvues de toute connexion audit support solide.

6. Méthode suivant la revendication 5, dans laquelle lesdites cibles libérées sont ensuite identifiées par hybridation à un microréseau en présence d'autres produits de ladite dégradation.

7. Méthode suivant l'une quelconque des revendications 1 à 6, dans laquelle on laisse lesdits ARNm présents dans ledit échantillon biologique s'hybrider avec lesdites cibles avant ledit couplage desdits ARNm audit support solide.

8. Méthode suivant l'une quelconque des revendications 1 à 7, dans laquelle, avant l'étape (f), lesdits ARNm couplés et lesdites cibles hybridées sont libérés dudit support solide.

9. Méthode suivant l'une quelconque des revendications 1 à 8, dans laquelle ledit support solide comprend une pluralité de billes magnétiques qui portent de l'oligod(T), et dans laquelle lesdites billes sont mélangées à l'échantillon biologique pour le couplage des ARNm à celles-ci.

10. Méthode suivant l'une quelconque des revendications 1 à 9, dans laquelle lesdites cibles sont des oligonucléotides d'ADN monocaténaire qui s'hybrident directement auxdits ARNm.

11. Méthode suivant l'une quelconque des revendications 1 à 10, dans laquelle lesdits ARNm sont dégradés par traitement alcalin dans l'étape (f) pour laisser lesdites cibles.

12. Méthode suivant la revendication 11, dans laquelle l'étape (g) est mise en oeuvre sans séparer lesdites cibles des résidus de l'étape de dégradation (f).

13. Méthode suivant l'une quelconque des revendications 1 à 12, dans laquelle lesdites cibles sont marquées initialement et lesdits marqueurs sont détectés dans l'étape (g).

14. Méthode suivant la revendication 13, dans laquelle, en tant que partie de l'étape (g), le produit de l'étape (f) est séparé dudit support solide et est hybridé avec un microréseau comprenant une pluralité de microtaches de caractère tridimensionnel, au moins une de ces microtaches contenant des sondes complémentaires de chacune desdites cibles.
